# EUROPÄISCHE PATENTSCHRIFT

(11) **EP 0 405 234 B1**
(45) Veröffentlichungstag und Bekanntmachung des Hinweises auf die Patenterteilung: **02.02.1994**
(21) Anmeldenummer: 90111214.4
(22) Anmeldetag: 13.06.1990
(51) Int. Cl.: A61B 6/00

(54) **Mobiles Röntgengerät**
Mobile X-ray apparatus
Appareil radiologique mobile

(30) Priorität: 28.06.1989 SE 8902332
(43) Veröffentlichungstag der Anmeldung: 02.01.1991
(73) Patentinhaber: Siemens-Elema AB, 171 95 Solna 1 (SE); SIEMENS AKTIENGESELLSCHAFT, 80333 München (DE)
(72) Erfinder: Warden, Hans, Ing., S-194 51 Upplands Väsby (SE); Westergren, Kent, Ing., S-175 40 Järfälla (SE)

(56) Entgegenhaltungen:
- US-A- 3 790 805
- US-A- 4 223 230
- US-A- 4 326 131

## Beschreibung

Die Erfindung betrifft ein mobiles Röntgengerat mit einer Röntgenröhre, die über eine Armvorrichtung an einem Wagen befestigt ist, der mit Rädern versehen und auf dem Boden fahrbar ist, wobei die Röntgenröhre in der Längsrichtung des Wagens und in der Höhe verstellbar ist.

Ein mobiles Röntgengerät dieser Art ist durch den Siemens-Prospekt Mobilett II bekannt. Die Armvorrichtung besteht aus einem Armteil, an dem die Röntgenröhre befestigt ist, und der mit einem zweiten Armteil, der an der Oberseite des Wagens angebracht ist, schwenkbar verbunden ist. Der Wagen ist mit einem Ausleger versehen, der als Stütze dient, wenn die Röntgenröhre in ihre Längsrichtung bzw. in der Höhe eingestellt wird.

Bei einer Röntgenaufnahme wird das mobile Röntgengerät zu einem bettlägrigen Patienten gefahren. Bei der Positionierung der Röntgenröhre ist sehr häufig eine Längs- und eine Seitenverschiebung der Röntgenröhre erforderlich.

Der Erfindung liegt die Aufgabe zugrunde, ein mobiles Röntgengerät der eingangs genannten Art zu schaffen, bei dem eine Positionierung der Röntgenröhre in einer raschen und einfachen Weise vorgenommen werden kann.

Diese Aufgabe ist erfindungsgemäß dadurch gelöst, daß am Wagen eine zum Boden absenkbare derart gelagerte Abstützvorrichtung angeordnet ist, daß diese in ihrer abgesenkten Lage das Röntgengerät so abstützt, daß dieses um die Abstützvorrichtung drehbar ist. Durch die Erfindung kann die Röntgenröhre einfach positioniert werden gleichzeitig damit, daß der Wagen in eine Position gebracht wird, in der er bei einer Drehung nicht gegen ein Bett oder einen anderen Gegenstand stößt.

Es ist zwar durch die US-A-3 790 905 bekannt, daß eine Röntgenröhre eines mobilen Röntgengerätes in seiner Längsrichtung und seitlich verschiebbar ist, ohne den Wagen des Röntgengerätes zu bewegen. Die Röntgenröhre ist an einem horizontal angeordneten Teleskoparm befestigt, der an einer vertikalen Säule angebracht ist, die um ihre Achse drehbar und mit dem Wagen verbunden ist. Die Röntgenröhre kann hierdurch in solche Lagen positioniert werden, in denen der Schwerpunkt des Röntgengerätes erheblich versetzt wird. Dies führt mit sich, daß die Abstützfläche gegen den Boden in allen Richtungen verhältnismäßig groß sein muß, um zu verhindern, daß das Gerät umkippt. Mit einem solchen Röntgengerät ist es schwer, zwischen den Betten zu manövrieren.

In einer vorteilhaften Ausbildung der Erfindung wird vorgeschlagen, daß die Abstützvorrichtung das Röntgengerät derart anhebt, daß die Räder vom Boden abheben. Dadurch wird eine gegenüber dem Boden reibungsfreie Drehung des Röntengerätes erreicht.

Die Abstützvorrichtung kann auch so ausgebildet werden, daß sie die Räder derart entlastet, daß diese bei einer Drehung des Röntgengerätes um die Abstützvorrichtung als Bodenstützen dienen.

In einer weiteren Ausbildung der Erfindung wird vorgeschlagen, daß die Abstützvorrichtung mittels einer Antriebsvorrichtung in der Höhe verstellbar ist. Hierdurch können die Bewegungen der Abstützvorrichtung in einfacher Weise gesteuert werden.

In einer konstruktiv einfachen Ausgestaltung der Erfindung wird vorschlagen, daß die Abstützvorrichtung mit einer Abstützplatte versehen ist, die an dem einen Ende eines zweiteiligen Gelenkarmes angebracht ist, dessen anderes Ende mit dem Wagen drehbar verbunden ist. Der zweiteilige Gelenkarm kann mit Hilfe der Antriebsvorrichtung zusammengeklappt bzw. auseinandergeklappt werden, wodurch die Abstützplatte in der Höhe verstellbar ist.

Eine günstige Ausgestaltung der Erfindung ergibt sich, wenn die Abstützplatte mit einer Welle versehen ist, die an einem sphärischen Lager befestigt ist, das seinerseits am Lenkarm angebracht ist. Dadurch ist erreicht, daß die Abstützplatte sich auch an einen etwas unebenen Boden anpaßt.

Im Hinblick auf eine vorteilhafte Weiterbildung der Erfindung wird vorgeschlagen, daß die Abstützplatte an der Abstützvorrichtung federnd gelagert ist. Hierdurch wird erreicht, daß die Abstützplatte auch bei unterschiedlichen bzw. ungleichmäßigen Bodenbelägen überall mit annähernd gleichem Druck anliegt.

Die Erfindung ist nachfolgend anhand von mehreren, in den Zeichnungen dargestellten Ausführungsbeispielen näher erläutert. Es zeigen:
- FIG 1: eine Seitenansicht eines mobilen Röntgengerätes mit einer Abstützvorrichtung nach der Erfindung
- FIG 2: eine Draufsicht des in der FIG 1 dargestellten mobilen Röntgengerätes und
- FIG 3 und 4: Seitenansichten einer Abstützvorrichtung nach der Erfindung.

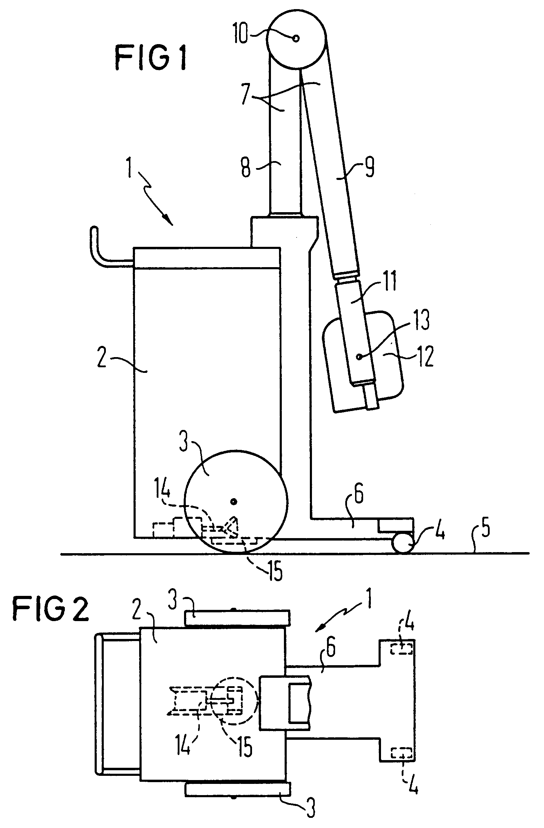

In der FIG 1 ist ein mobiles Röntgengerät (1) gezeigt mit einem Wagen (2), der mit zwei Laufrädern (3) und zwei Lenkrädern (4) versehen ist und der auf dem Boden (5) fahrbar ist. Die Lenkräder (4) sind an einem vom Wagen (2) ausgehenden Ausleger (6) befestigt. Das Röntgengerät (1) ist mit einem Doppelarm (7) versehen, dessen einer Armteil (8) an der Oberseite des Wagens (2) in einer etwa vertikalen Lage befestigt ist und dessen anderer Armteil (9) mit dem Armteil (8) um eine Achse (10) schwenkbar verbunden ist. Der Armteil (9) trägt über eine Halterung (11) eine Röntgenröhre (12), die um eine Achse (13) drehbar an der Halterung (11) befestigt ist. Die Röntgenröhre (12) wird in dieser FIG 1 in ihrer Parklage gezeigt. Der Ausleger (6) dient als Stütze für den Wagen (2), wenn der Armteil (9) mit der Röntgenröhre (12) um die Achse (10) in eine Arbeitslage geschwenkt wird. Im unteren Bereich des Wagens (2) zwischen den Laufrädern (3) ist eine Abstützvorrichtung (14) für das Röntgengerät (1) angeordnet. Die Abstützvorrichtung (14) wird später näher beschrieben.

In der FIG 2 ist dargestellt, wie die Abstützvorrichtung (14) zwischen den Laufrädern (3) angebracht ist.

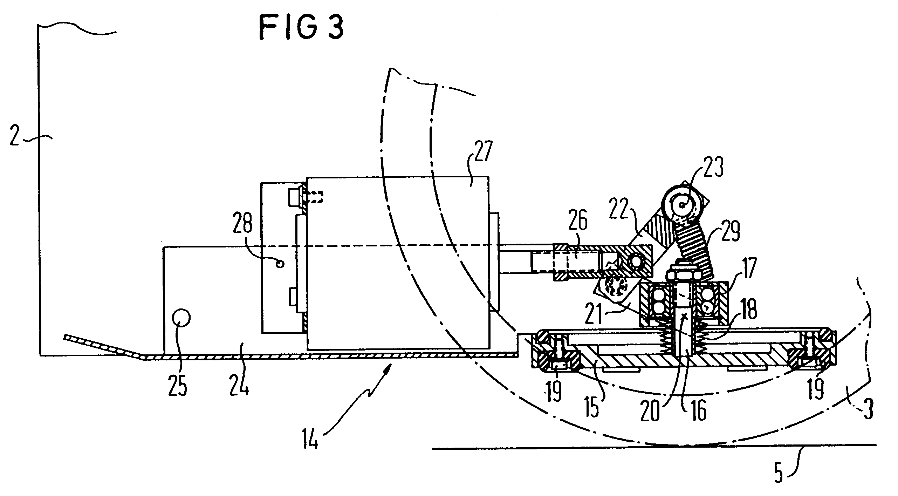

In der FIG 3 ist die Abstützvorrichtung (14) gezeigt, die mit einer Abstützplatte (15) mit einem Lagerbolzen (16) versehen ist, der in einem sphärischen Lager (17) schwenkbar gelagert ist. Die Abstützplatte (15) ist mittels einer Feder (18), die um den Lagerbolzen (16) zwischen der Abstützplatte (15) und dem Lager (17) angeordnet ist, federnd gelagert. Die untere Seite der Abstützplatte (15) ist außerdem mit einer Anzahl Gummistöpsel (19) versehen. Das Lager (17) ist um eine Achse (20) drehbar, die dieses Lager (17) mit dem freien Ende eines Armes (21) eines zweiteiligen Gelenkarmes (21,22) verbindet. Das freie Ende des Armes (22) des zweiteiligen Gelenkarmes (21,22) ist um eine Achse (23), die am Wagen (2) befestigt ist, drehbar. Die Achse (20) ist außerdem an einer Konsole (24) angebracht, die ihrerseits mittels einer Achse (25) mit dem Wagen (2) drehbar verbunden ist. Am Arm (22) des zweiteiligen Gelenkarmes (21,22) ist eine Antriebsstange (26) befestigt, die von einem Elektromagneten (27) angetrieben wird, der an der Konsole (24) mittels einer Achse (28) drehbar befestigt ist.

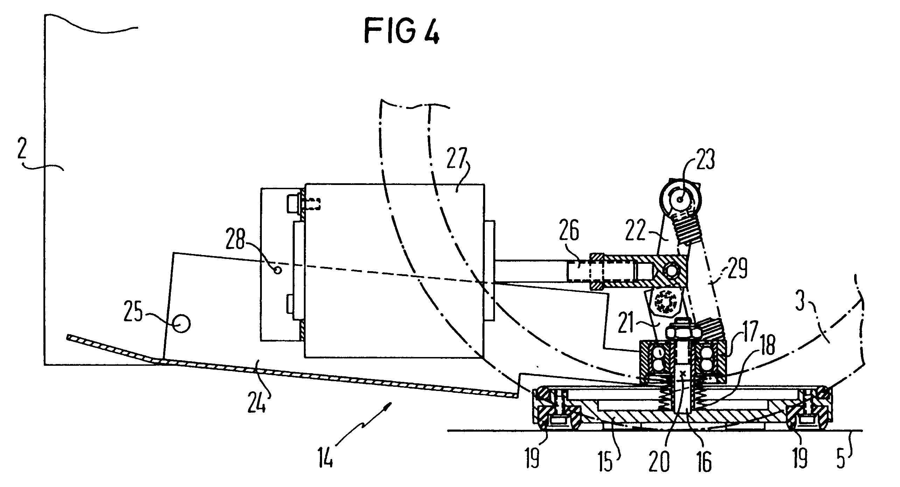

In der FIG 4 ist die Abstützvorrichtung (14) dargestellt, wenn sich die Abstützplatte (15) in ihrer abgesenkten Lage befindet. Diese Lage wird erreicht, wenn der Elektromagnet (27) aktiviert wird. Der Elektromagnet (27) verschiebt bei einer Aktivierung die Antriebsstange (26), die gegen den zweiteiligen Gelenkarm (21,22) drückt derart, daß der Gelenkarm (21,22) ausgerichtet wird. Gleichzeitig dreht sich die Konsole (24) zwangsläufig um die Achse (25). Wenn die Abstützplatte (15) sich in ihrer abgesenkten Lage befindet, liegt sie im Zentrum zwischen den Laufrädern (3), wie es in der FIG 2 gezeigt ist. Wenn nun die Abstützplatte (15) mit Hilfe des sphärischen Lagers (17) und der Feder (18) dicht am Boden (5) liegt und die Laufräder (3) entlastet, kann das Röntgengerät (1) leicht um die Abstützplatte (15) gedreht werden, wobei die Laufräder (3) als Bodenstütze dienen. Durch diese Arretierung des Röntgengerätes (1) gleichzeitig damit, daß es drehbar ist, kann eine Positionierung der Röntgenröhre (12) in einer schnellen und einfachen Weise erfolgen.

Wenn die Röntgenuntersuchung beendet ist, wird der Strom zum Elektromagneten (27) ausgeschaltet, wobei eine Zugfeder (29), deren eines Ende an der Achse (23) und deren anderes Ende an der Konsole angebracht ist, den zweiteiligen Gelenkarm (21,22) zusammenklappt, so daß die Antriebsstange (27) in den Elektromagneten (27) hineingeschoben wird und die Abstützplatte (15) in ihre Ausgangslage zurückgeht. Als Antrieb für die Abstützvorrichtung (14) kann der Elektromagnet (27) mit der Antriebsstange (26) z.B. mit einem elektrischen Motor mit einer Spindel ersetzt werden.

In einer weiteren Ausführungsform, die nicht dargestellt ist, kann die Abstützvorrichtung (14) das Röntgengerät (1) derart heben, daß auch die Räder (3,4) vom Boden frei gehen. In diesem Ausführungsbeispiel ist es nicht notwendig, daß die Abstützplatte (15) in ihrer abgesenkten Lage im Zentrum zwischen den Laufrädern (3) liegt.

## Patentansprüche

1. Mobiles Röntgengerät mit einer Röntgenröhre, die über eine Armvorrichtung an einem Wagen befestigt ist, der mit Rädern versehen und auf dem Boden fahrbar ist, wobei die Röntgenröhre in der Längsrichtung des Wagens und in der Höhe verstellbar ist, **dadurch gekennzeichnet,** daß am Wagen (2) eine zum Boden (5) absenkbare derart gelagerte Abstützvorrichtung (14) angeordnet ist, daß diese in ihrer abgesenkten Lage das Röntgengerät (1) so abstützt, daß dieses um die Abstützvorrichtung (14) drehbar ist.

2. Mobiles Röntgengerät nach Anspruch (1), **dadurch gekennzeichnet,** daß die Abstützvorrichtung (14) das Röntgengerät (1) derart anhebt, daß die Räder (3,4) vom Boden (5) frei gehen.

3. Mobiles Röntgengerät nach Anspruch (1), **dadurch gekennzeichnet,** daß die Abstützvorrichtung (14) die Räder (3,4) derart entlastet, daß diese bei einer Drehung des Röntgengerätes (1) um die Abstützvorrichtung (14) als Bodenstützen dienen.

4. Mobiles Röntgengerät nach einem der Ansprüche 1 bis 3, **dadurch gekennzeichnet,** daß die Abstützvorrichtung (14) mittels einer Antriebsvorrichtung in der Höhe verstellbar ist.

5. Mobiles Röntgengerät nach Anspruch 4, **dadurch gekennzeichnet,** daß als Antriebsvorrichtung ein Elektromagnet (27) mit einer Antriebsstange (26) verwendet wird.

6. Mobiles Röntgengerät nach Anspruch 4, **dadurch gekennzeichnet,** daß als Antriebsvorrichtung (26,27) ein elektrischer Motor mit einer Gewindestange verwendet wird.

7. Mobiles Röntgengerät nach einem der Ansprüche 1 bis 6, **dadurch gekennzeichnet**, daß die Abstützvorrichtung (14) mit einer Abstützplatte (15) versehen ist, die an dem einen Ende eines zweiteiligen Gelenkarmes (21,22) angebracht ist, dessen anderes Ende mit dem Wagen (2) drehbar verbunden ist, und daß der zweiteilige Gelenkarm (21,22) mit Hilfe der Antriebsvorrichtung (26,27) zusammenklappbar bzw. auseinanderklappbar ist, wodurch die Abstützplatte (15) in der Höhe verstellbar ist.

8. Mobiles Röntgengerät nach einem der Ansprüche 1 bis 7, bei dem der Wagen (2) mit mindestens zwei Laufrädern (3) und zwei Lenkrädern (4) versehen ist, **dadurch gekennzeichnet**, daß die Abstützplatte (15) in der abgesenkten Lage im Zentrum zwischen den Laufrädern (3) liegt.

9. Mobiles Röntgengerät nach einem der Ansprüche 1 bis 8, **dadurch gekennzeichnet**, daß die Abstützplatte (15) mit einer Welle (16) versehen ist, die an einem sphärischen Lager (17) befestigt ist, das seinerseits am Lenkraum (21,22) angebracht ist.

10. Mobiles Röntgengerät nach einem der Ansprüche 1 bis 9, **dadurch gekennzeichnet**, daß die Abstützplatte federnd an der Abstützvorrichtung gelagert ist.

## Claims

1. Mobile X-ray machine having an X-ray tube fastened by an arm device to a trolley, which trolley is provided with wheels and can be transported on the floor, the X-ray tube being adjustable in the longitudinal direction of the trolley and in height, characterized in that on the trolley (2) there is disposed a supporting device (14) which can be lowered to the floor (5) and is mounted in such a way that the said supporting device, in its lowered position, supports the X-ray machine (1) in such a way that the latter is rotatable about the supporting device (14).

2. Mobile X-ray machine according to Claim 1, characterized in that the supporting device (14) raises the X-ray machine (1) in such a way that the wheels (3, 4) move free from the floor (5).

3. Mobile X-ray machine according to Claim 1, characterized in that the supporting device (14) relieves the wheels (3, 4) of load in such a way that they serve as floor supports when the X-ray machine (1) is rotated about the supporting device (14).

4. Mobile X-ray machine according to one of Claims 1 to 3, characterized in that the supporting device (14) can be adjusted in height by means of a drive device.

5. Mobile X-ray machine according to Claim 4, characterized in that, as the drive device, an electromagnet (27) with a drive rod (26) is used.

6. Mobile X-ray machine according to Claim 4, characterized in that, as the drive device (26, 27), an electric motor with a threaded rod is used.

7. Mobile X-ray machine according to one of Claims 1 to 6, characterized in that the supporting device (14) is provided with a supporting plate (15) which is fitted to the one end of a two-part articulated arm (21, 22), the other end of which is connected rotatably to the trolley (2), and in that the two-part articulated arm (21, 22) can be folded together or folded apart with the aid of the drive device (26, 27), thereby enabling the supporting plate (15) to be adjusted in height.

8. Mobile X-ray machine according to one of Claims 1 to 7, in which the trolley (2) is provided with at least two running wheels (3) and two direction-steering wheels (4), characterized in that the supporting plate (15) lies, in the lowered position, in the centre between the running wheels (3).

9. Mobile X-ray machine according to one of Claims 1 to 8, characterized in that the supporting plate (15) is provided with a shaft (16) fastened to a spherical bearing (17), which, for its part, is fitted to the articulated arm (21, 22).

10. Mobile X-ray machine according to one of Claims 1 to 9, characterized in that the supporting plate is mounted resiliently on the supporting device.

## Revendications

1. Appareil radiologique mobile-comportant un tube à rayons X, qui est fixé au moyen d'un dispositif à bras sur un chariot qui est pourvu de roues et peut être déplacé sur le sol, le tube à rayons X étant réglable dans la direction longitudinale du chariot et en hauteur, caractérisé par le fait que sur le chariot (2) est prévu un dispositif de support (14), qui est monté de manière à pouvoir être abaissé par rapport au sol (5) de telle sorte que, dans sa position abaissée, ce dispositif de support supporte l'appareil radiologique (1) de manière que ce dernier puisse être entraîné en rotation autour du dispositif de support (14).

2. Appareil radiologique mobile suivant la revendication 1, caractérisé par le fait que le dispositif de support (14) soulève l'appareil radiologique (1) de manière que les roues (3,4) s'écartent du sol (5).

3. Appareil radiologique mobile suivant la revendication 1, caractérisé par le fait que le dispositif de support (14) décharge les roues (3,4) de telle sorte que, lors d'une rotation de l'appareil radiologique (1) autour du dispositif de support (14), ces roues sont utilisées en tant qu'appuis au sol.

4. Appareil radiologique mobile suivant l'une des revendications 1 à 3, caractérisé par le fait que le dispositif de support (14) est réglable en hauteur au moyen d'un dispositif d'entraînement.

5. Appareil radiologique mobile suivant la revendication 4, caractérisé par le fait qu'on utilise comme dispositif d'entraînement un électroaimant (27) possédant une barre d'entraînement (26).

6. Appareil radiologique mobile suivant la revendication 4, caractérisé par le fait qu'on utilise comme dispositif d'entraînement un moteur électrique possédant une tige filetée.

7. Appareil radiologique mobile suivant l'une des revendications 1 à 6, caractérisé par le fait que le dispositif de support (14) est pourvu d'une plaque de support (15), qui est montée sur une extrémité d'un bras articulé (21,22) formé de deux éléments et dont l'autre extrémité est reliée au chariot (2), de manière à pouvoir tourner, et que le bras articulé (21,22) formé de deux éléments peut être rétracté ou déployé par rabattement à l'aide du dispositif d'entraînement (26,27), ce qui permet de régler en hauteur la plaque de support (15).

8. Appareil radiologique mobile suivant l'une des revendications 1 à 7, dans lequel le chariot (2) est équipé d'au moins deux roues (3) et de deux roues orientables (4), caractérisé par le fait que la plaque de support (15) est située, dans la position abaissée, en position centrée entre les roues (3).

9. Appareil radiologique mobile suivant l'une des revendications 1 à 8, caractérisé par le fait que la plaque de support (15) comporte un arbre (16), qui est fixé sur un palier sphérique (17) qui, pour sa part, est monté sur le bras articulé (21,22).

10. Appareil radiologique mobile suivant l'une des revendications 1 à 9, caractérisé par le fait que la plaque de support est supportée élastiquement sur le dispositif de support.
